Europäisches Patentamt

(19) European Patent Office

Office européen des brevets

(11) Veröffentlichungsnummer: **0 158 310**

**A2**

(12) **EUROPÄISCHE PATENTANMELDUNG**

(21) Anmeldenummer: 85104223.4

(22) Anmeldetag: 06.04.85

(51) Int. Cl.⁴: **H 05 F 7/00**
A 61 J 3/00, A 61 N 1/16

(30) Priorität: 11.04.84 DE 3413540

(43) Veröffentlichungstag der Anmeldung:
16.10.85 Patentblatt 85/42

(84) Benannte Vertragsstaaten:
AT CH DE FR LI NL

(71) Anmelder: Terwen, Peter sen.
Allee 1
D-7100 Heilbronn(DE)

(71) Anmelder: Nonnenbroich, Friedhelm
Allee 1
D-7100 Heilbronn(DE)

(72) Erfinder: Terwen, Peter, Sr.
Hofgartenstrasse 21/1
D-7100 Heilbronn(DE)

(72) Erfinder: Nonnenbroich, Friedhelm
Allee 1
D-7100 Heilbronn(DE)

(74) Vertreter: Jeser, Jean-Pierre
Lerchenstrasse 56
D-7100 Heilbronn(DE)

(54) Übertragungsgerät für Elektroakupunkturwirkungen.

(57) Ein Übertragungsgerät für Medikamentenwirkung bei Elektroakupunkturverfahren weist eine Aufstellfläche (18) und eine mit dieser über zwei parallele Verbindungsleitungen (27) verbundene Empfangsfläche (19) auf. An die Verbindungsleitungen ist eine einstellbare Spannungsquelle (28) angeschlossen, durch die ein geringer Stromfluß durch die Verbindungsleitungen einstellbar ist. Bei fließendem Strom wird Information von einer auf die Aufstellfläche aufgestellten Substanz auf eine auf die Empfangsfläche (19) aufgebrachte Substanz mit umgekehrtem Vorzeichen übertragen. Dadurch kann die Krankheiten hervorrufende Information eines Schadstoffes direkt in Heilinformation auf der zweiten Substanz, z. B. einem Kunststoffstreifen (30) umgewandelt werden. Der Kunststoffstreifen wird dann auf eine Akupunkturstelle aufgebracht, die einem Organ zugeordnet ist, das durch Einfluß des Schadstoffes erkrankt ist.

Fig.5

EP 0 158 310 A2

- 1 -

BESCHREIBUNG


Übertragungsgerät für Elektroakupunkturwirkungen


TECHNISCHES GEBIET·


Die Erfindung betrifft ein Übertragungsgerät für Elektroakupunkturwirkungen.


STAND DER TECHNIK

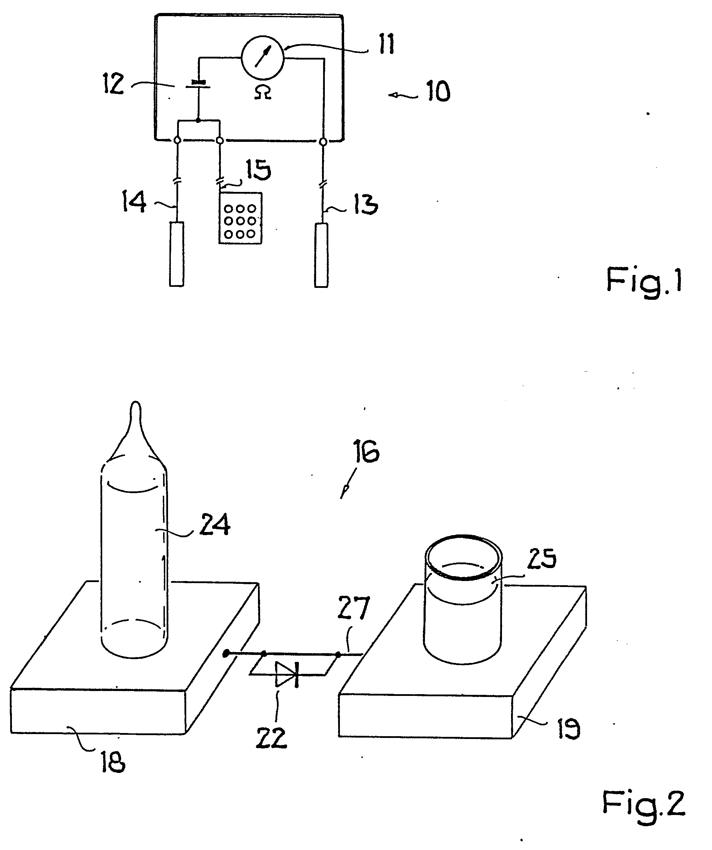

Der sogenannte Voll'sche Medikamententest ⌐anhand von⌐ Elektroakupunkturverfahren dient dazu, aus einer Vielzahl von Medikamenten ein besonders geeignetes zum Behandeln einer Krankheit auszusuchen. Der Test wird mit Geräten durchgeführt, wie sie seit Jahren handelsüblich sind. Die Medikamente werden in Ampullen von der Arzneimittelindustrie geliefert. Das Meßverfahren wird im folgenden ausgehend von einem Meßgerät beschrieben, wie es in Fig. 1 dargestellt ist.

Das Meßgerät 10 gemäß Fig. 1 weist ein Ohmmeter 11, eine Gleichspannungsquelle 12, eine Meßsonde 13, eine Handelektrode 14 und eine sogenannte Medikamentenwabe 15 auf. Die Medikamentenwabe 15 und die Handelektrode 14 sind an den negativen Pol der Gleichspannungsquelle 12 angeschlossen. Der positive Pol der Gleichspannungsquelle steht mit einem Eingang des Ohmmeters 11 in Verbindung, während der andere Eingang an die Meßsonde 13 angeschlossen ist.

Mit diesem Gerät geht eine Messung wie folgt vor sich. Eine zu untersuchende Person nimmt die Handelektrode 14 in eine Hand. Der Arzt tastet dann mit der stiftförmigen Meßsonde 13 Akupunkturpunkte am Körper der zu untersuchenden Person ab. Es ist in der Medizin bekannt, daß Zusammenhänge zwischen bestimmten Erkrankungen und dem Verhalten bestimmter Akupunkturpunkte bestehen. Mit dem angegebenen Gerät wird der Körperwiderstand zwischen der Handelektrode 14 und dem durch die Meßsonde 13 ausgewählten Akupunkturpunkt gemessen. Ist das dem Akupunkturpunkt zugeordnete Organ gesund, so tritt ein bestimmter, bekannter Widerstand auf. Das Gerät ist so kalibriert, daß es dann den Wert 0 anzeigt. Ist das dem Akupunkturpunkt zugeordnete Organ in seiner Leistungsfähigkeit dagegen geschwächt, oder weist es übersteigerte Leistungsfähigkeit auf, so schlägt das Ohmmeter nach der einen bzw. der anderen Seite aus. Durch den Ausschlag des Meßgerätes wird also festgestellt, ob das dem kontrollierten Akupunkturpunkt zugeordnete Organ geschwächt, gesund oder überstark ist. Der Zusammenhang zwischen Krankheitszuständen und gemessenen Werten ist z. B. in der Zeitschrift "Physikalische Medizin und Rehabilitation" 1980, S. 160 - 162 beschrieben.

Die genannte Messung läßt sich dadurch verändern, daß ein Medikament, das von der pharmazeutischen Industrie zu diesem Zweck in eine Ampulle verpackt geliefert wird, in die Medikamentenwabe 15 gestellt wird. Es fällt auf, daß zwischen der Meßsonde 13 und der Medikamentenwabe 15 keine ohmsche Verbindung besteht. Es wurde jedoch gezeigt, daß dennoch der vom Ohmmeter 11 angezeigte Wert beeinfluß wird. Die Beeinflussung hängt vom jeweils gewählten Medikament ab. Dies ist z. B. in der "Ärztezeitschrift für Naturheilverfahren", 1981, Heft 6 durch E. Höllischer und W. Mehlhardt beschrieben.

Beim Meßgerät 10 gemäß Fig. 1 wirkt also die Medikamentenwabe 15 als Übertragungsgerät für Medikamentenwirkung , wie sie durch ein Elektroakupunkturverfahren feststellbar ist. Die Wabe 15 weist

0158310

- 3 -

eine Aufstellfläche zum Aufstellen der verschiedenen Medikamente auf.

Die Auswahl eines Medikamentes läuft mit der Meßeinrichtung 10 gemäß Fig. 1 wie folgt ab. Nach dem oben angegebenen Verfahren wird z. B. festgestellt, daß die Funktion eines bestimmten Organes einer untersuchten Person geschwächt ist. Dies gibt einen Ausschlag auf dem Ohmmeter 11, der im folgenden als negativ bezeichnet wird. Der Arzt gibt dann eine Ampulle mit einem Medikament in die Medikamentenwabe 15 und mißt dann den Körperwiderstand zwischen der Handelektrode 14 und der Meßsonde 13, die am zugehörigen Akupunkturpunkt aufgesetzt wird, wieder. Diese Prozedur wird solange vollzogen, bis das Ohmmeter wieder auf null steht. Dann ist das richtige Medikament in der richtigen Menge oder die richtige Medikamentenzusammenstellung ausgewählt. Das Medikament oder die Medikamente werden dem Patienten dann gespritzt oder er nimmt sie ein.

## DIE ERFINDUNG UND DEREN WIRKUNGEN

Das erfindungsgemäße Übertragungsgerät für Elektroakupunkturwirkungen zeichnet sich dadurch aus, daß es eine Eingangsseite und eine Ausgangsseite aufweist, die jeweils aus einem elektrisch leitfähigen Material bestehen und die über mindestens ein elektrisch leitendes Verbindungsstück miteinander verbunden sind. Kommt mit der Eingangsseite ein Gegenstand in Berührung, der eine bestimmte Elektroakupunkturwirkung in obigem Sinne ausübt, so wird diese Wirkung auf die Ausgangsseite übertragen. Liegt eine ungeradzahlige Anzahl von Verbindungsstücken vor, wird die Wirkung, die der Gegenstand auf der Eingangsseite ausübt, zur Ausgangsseite hin umgedreht. Liegt dagegen eine gradzahlige Anzahl von Verbindungsstücken vor, wird die Wirkung umgedreht. Die unveränderte Weitergabe der Information kann dazu ausgenutzt werden, daß die Wirkung eines Medikamentes auf eine andere Substanz übertragen wird, was besonders dann von Vorteil ist, wenn das Me-

0158310

- 4 -

dikament sehr teuer ist. Vom Patienten kann dann die billige Substanz, auf die die Medikamentenwirkung übertragen wurde, zur Heilung verwerdern. Wird dagegen ein Gerät verwendet, bei dem die Wirkung umgedreht wird, ist es möglich, daß ein Patient die rechte Hand auf die Eingangsseite und die linke Hand auf die Ausgangsseite legt. Auf die linke Hand wirkt dann eine Wirkung, die zu der von der rechten Hand ausgeübten Wirkung umgekehrt ist. Dies führt zu einer Neutralisierung der Wirkungen. Eine entsprechende Neutralisierung ist auch möglich, wenn ein derartiges Gerät z.B. im Fahrgastraum eines Kraftfahrzeuges aufgestellt wird. Sind im Fahrgastraum Wirkungen vorhanden, die sich derart bemerkbar machen, daß Organe der Personen im Fahrgastraum belastet werden, was wie eingangs erläutert gemessen wird, so werden diese Wirkungen von der Eingangsseite des Übertragungsgerätes erfaßt, und von einer Antennte auf der Ausgangsseite wird ein Signal abgegeben, das eine belebende Funktion auf die Organe ausübt. Dadurch findet wieder ein Neutralisieren der belastenden Wirkungen statt. So wurde z.B. festgestellt, daß bei Fahrern von Kraftfahrzeugen der Pulsschlag bei etwa 120 Schlägen pro Minute liegt und in Streßsituationen auf 150 Schläge pro Minute steigen kann. Bei Verwendung eines erfindungsgemäßen Übertragungsgerätes im Fahrgastraum beträgt der der Pulsschlag dagegen nur 90 Schläge pro Minute. Die genannte Neutralisierung ist weiterhin zum Behandeln von Speisen anwendbar. Um beim Kochen von Speisen auf diese übergegangene Fremdwirkungen zu kompensieren, werden die Speisen auf die Ausgangsseite des Gerätes gestellt.

KURZE BESCHREIBUNG DER ZEICHNUNGEN

Fig. 1  schematische Darstellung eier bekannten Meßeinrichtung mit einem Übertragungsgerät für Medikamentenwirkung;

Fig. 2    perspektivische Ansicht einer anmeldegemäßen Ausführungsform zum Übertragen der Wirkung eines Medikamentes auf eine andere Substanz;

Fig. 3    perspektivische Ansicht einer weiteren
Ausführungsform, zum umkehrenden Übertragen
der Wirkung einer Substanz;

Fig. 4    Schnitt durch das Gerät gemäß Fig. 3 entlang der Linie 3-3; und

Fig. 5    perspektivische Ansicht mit einem schematischen Schaltbild einer steuerbaren Ausführungsform.

Fig. 6    perspektivische Teilansicht eines in einem
Fahrgastraum aufstellbaren Gerätes; und

Fig. 7    schematische Teilansicht von unten auf den
Deckel des Gerätes von Fig. 6.

WEGE ZUM AUSFÜHREN DER ERFINDUNG

Die Ausführungsform eines anmeldegemäßen Übertragungsgerätes 16 gemäß Fig. 2 weist eine plattenförmige Aufstellfläche 18 als Eingangsseite und eine plattenförmige Empfangsfläche 19 als Ausgangsseite auf. Die Eingangsseite und die
Ausgangsseite sind über ein Verbindungsstück miteinander
verbunden, das als elektrische Verbindungsleitung 27 mit
parallel liegender Diode 22 ausgebildet ist. Die Diode 22
läßt von der Aufstellfläche 18 zur Empfangsfläche 19 durch.
Die plattenförmige Aufstellfläche 18 und die plattenförmige
Empfangsfläche 19 bestehen jeweils aus Metallplatten, z. B.
aus Aluminium oder Messing.

Auf die Aufstellfläche 18 ist eine Medikamentenampulle 24
aufgestellt, die ein Medikament enthält, das mit dem Medikamententest zum Behandeln einer Krankheit ausgesucht worden ist, wie dies an Hand von Fig. 1 oben beschrieben ist. Auf die Empfangsfläche 19 ist ein Substanzbehälter 25 aufgesetzt, in dem sich eine billige Substanz wie z. B. Zucker befindet. Durch das Übertragungsgerät 16 wird die Heilinformation vom Medikament in der Medikamentenampulle 24 auf die billige Substanz im Substanzbehälter 25 übertragen. Die zu behandelnde Person nimmt dann die billige Substanz aus dem Substanzbehälter 25 ein, so daß das teure Medikament in der Medikamentenampulle 24 nicht verbraucht werden muß, sondern für weitere Medikamententests zur Verfügung steht.

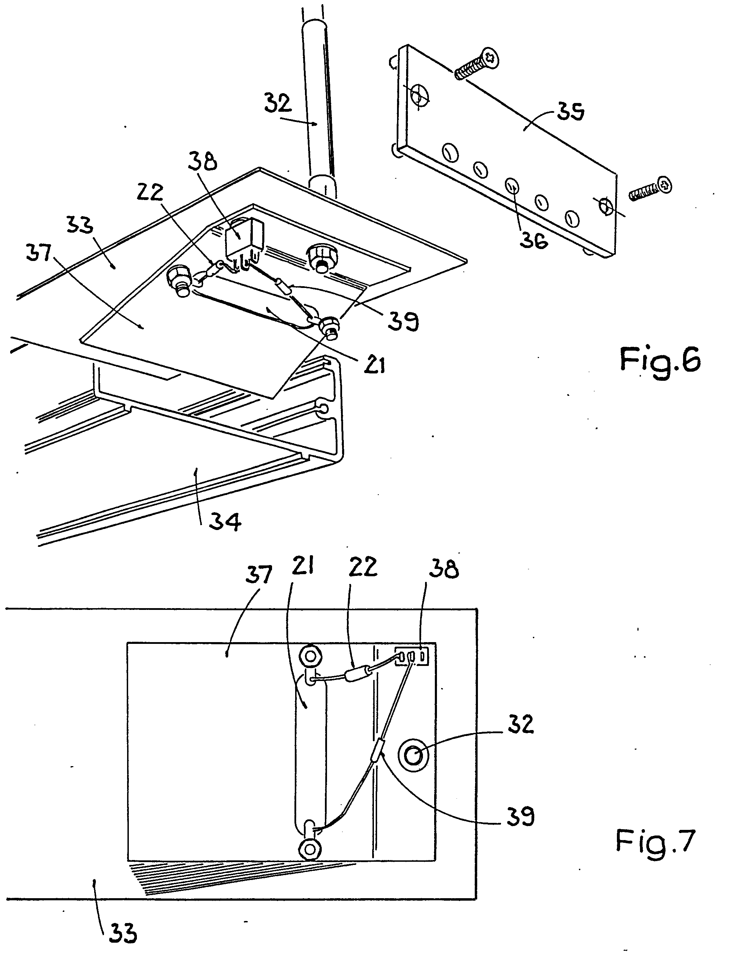

Statt dem Substanzbehälter 25 mit einer festen Substanz kann auch eine Ampulle mit einer flüssigen Substanz, z. B. einer physiologischen Kochsalzlösung auf die Empfangsfläche 19 aufgestellt oder aufgelegt sein. Die Information kann auch auf andere Möglichkeiten von der Empfangsfläche 19 aus weiter übertragen werden, wie dies weiter unten an Hand von Fig. 5 erläutert ist.

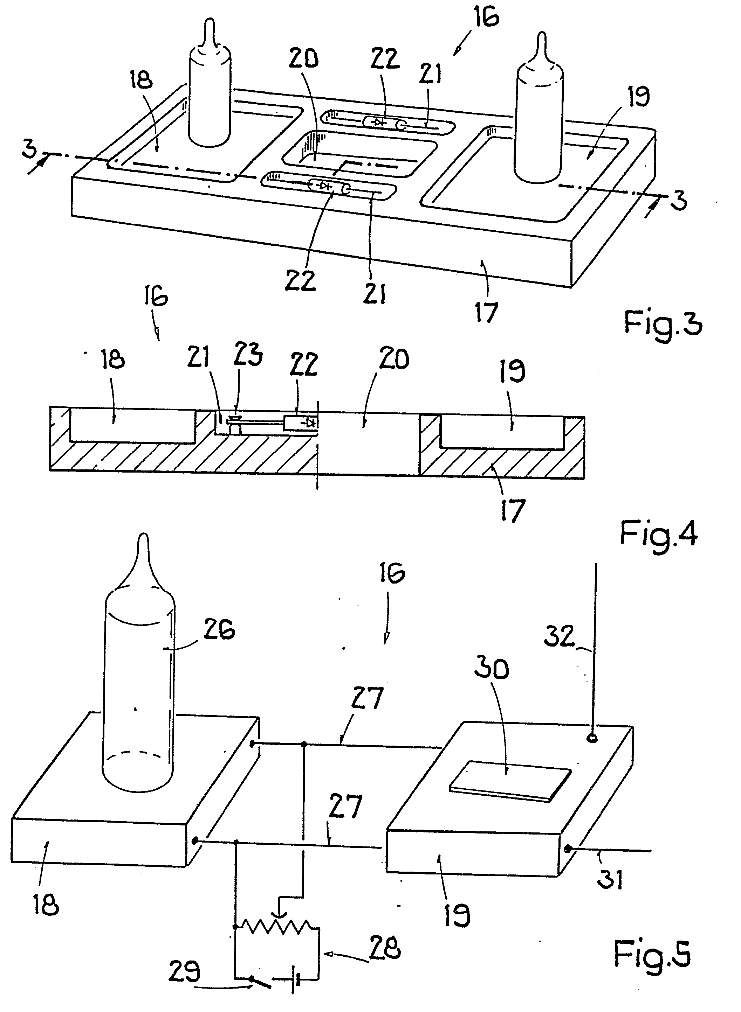

Die Aufstellfläche 18 und die Empfangsfläche 19 können auch strukturiert sein, um Ampullen oder andere Behälter besser haltern zu können. Die beiden Flächen können auch einstückig miteinander verbunden sein, wie dies im folgenden an Hand der Ausführungsform von Fig. 2 näher erläutert wird. In diesem Fall liegt die Diode 22 in einer schlitzförmigen Ausnehmung, ebenso wie die Dioden 22 bei der Ausführungsform gemäß Fig. 3 in schlitzförmigen Ausnehmungen 21 liegen. Irgendeine Strukturierung der Aufstellfläche 18 oder der Empfangsfläche 19 ist für die Funktion unerheblich. Die Strukturierung kann daher frei so gewählt werden, daß aufzubringende Substanzen am besten gehaltert werden.

Das Übertragungsgerät 16 gemäß Fig. 3 besteht aus einem Aluminiumklotz 17, der von oben her zwei Ausnehmungen aufweist, von denen die linke als Aufstellfläche 18 und die rechte als Empfangsfläche 19 wirkt. Zwischen den beiden Ausnehmungen 18 und 19 ist eine Durchgangsbohrung 20 vorhanden. Zu beiden Seiten der Durchgangsbohrung 20 ist jeweils eine schlitzförmige Ausnehmung 21 vorhanden, in denen jeweils eine Diode 22 so angeordnet ist, daß sie die Seite der Aufstellfläche 18 mit der Seite der Empfangsfläche 19 in Durchgangsrichtung verbindet. Die noch stehenden Bereiche des Aluminiumklotzes 17 um die schlitzförmigen Ausnehmungen 21 herum dienen als direkte ohmsche, zueinander parallele Verbindungen zwischen der Aufstellfläche 18 und der Empfangsfläche 19. Zu diesen ohmschen Verbindungen liegen die Dioden 22 parallel. Die Dioden 22 sind zum Beispiel durch eine Klemmverbindung 23 am Klotz 17 befestigt, wie sie in Fig. 4 dargestellt ist.

Diese Anordnung wirkt nicht mehr direkt übertragend wie die Anordnung gemäß Fig. 2, sondern sie dreht die Wirkung der auf die Aufstellfläche 18 aufgestellten Substanz um. Bei diesem Übertragungsgerät 16 ist es daher nicht mehr erforderlich, links ein Medikament und rechts die billige Substanz aufzustellen, also zunächst das Medikamententestverfahren durchzuführen, um das richtige Medikament zu finden, sondern es kann, wenn bekannt ist, daß eine bestimmte Substanz einem Patienten schadet, z. B. ein bestimmter Kunststoff, direkt diese Schadsubstanz links aufgesetzt werden. Das Gerät kehrt die Schadwirkung dann in eine gegenteilige Wirkung um, die in die billige Substanz auf der Empfangsfläche 19 umgewandelt wird. Das Behandeln kann dann direkt mit der billigen Substanz erfolgen. Bei der Darstellung gemäß Fig. 3 befindet sich die billige Substanz in einem als Ampulle ausgebildeten Behälter 25, und auf die Aufstellfläche 18 ist Schadstoff in einer Schadstoffampulle 26 aufgesetzt. Die programmierte billige Substanz kann dann, wenn sie z.B. Zucker ist, eingenommen oder, wenn sie z.B. eine Kunststoffolie ist, auf einen aus dem Schadstoff hergestellten Gegenstand geklebt werden, um dessen Schadeinfluß zu kompensieren.

- 8 -

Die Geräte gemäß den Fig. 2 und 3 weisen einen Nachteil dahingehend auf, daß sie nicht steuerbar sind. So ist es z. B. wünschenswert, insbesondere dann, wenn die Information von einem Schadstoff in heilende Information umgewandelt wird, den Umwandlungsprozeß nach einer bestimmten Zeit, z. B. 5 Sekunden, 20 Sekunden oder einer Minute abzubrechen, um jeweils Heilsubstanzen bestimmter Wirkung zu erhalten. Ein solches Steuern ist mit der Anordnung gemäß Fig. 5 möglich.

Beim Übertragungsgerät 16 gemäß Fig. 5 liegen wiederum eine plattenförmige Aufstellfläche 18 und eine plattenförmige Empfangsfläche 19 vor. Diese sind über zwei zueinander parallele elektrische Verbindungsleitungen 27 miteinander verbunden. Diese Verbindungsleitungen 27 sind an eine einstellbare Spannungsquelle 28 angeschlossen. Die Spannungsquelle liefert von etwa 4,5 V einen Strom von etwa 1 - 30 μA. Je nach Stärke des Stromflusses erfolgt das Übertragen von der Aufstellfläche 18 zur Empfangsfläche 19 schneller oder langsamer, und zwar zunehmend schnell mit zunehmender Stromstärke. Um den Stromfluß unterbrechen und damit das Übertragen abbrechen zu können, weist die einstellbare Spannungsquelle 28 einen Schalter 29 auf. Dieser kann auch durch eine Zeituhr gesteuert sein. Dann kann durch Einstellen der Spannung der einstellbaren Spannungsquelle 28 und der Wirkungsdauer mit Hilfe des Schalters 29 genau vorgegeben werden, wie stark eine auf die Empfangsfläche 19 gelegte Substanz programmiert werden soll.

Bei der in Fig. 5 dargestellten Ausführungsform ist auf die Empfangsfläche 19 ein Streifen 30 eines Magnetbandes 30 gelegt. Dieses Magnetband 30 wird durch die übertragene Information programmiert und nach dem Programmieren wird es mit einem Klebestreifen am entsprechenden Akupunkturpunkt befestigt, der dem erkrankten Organ zugeordnet ist,
gegen dessen Erkrankung vorgegangen werden soll.

                              16
Mit einem  Übertragungsgerät /gemäß den Fig. 2-5 ist es also
möglich, die Heil- oder Krankheitsinformation einer ersten
Substanz auf eine zweite, billige Substanz zu übertragen.


Das Material für die Aufstellfläche 18 und die Empfangsfläche 19 kann aus einem beliebigen elektrisch leitfähigen Material gewählt sein. Es muß nicht für beide Flächen
dasselbe Material verwendet sein. Es kommt nicht darauf
an, ob die Flächen eben sind, wie bei den Ausführungsformen gemäß den Fig. 2 und 5, oder ob sieAusnehmungen aufweisen, wie die Ausführungsformen gemäß Fig. 3.

Auch die Abmessungen des Gerätes spielen für seine Funktion keine Rolle. Elektrische Verbindungsleitungen einschließlich der Dioden können an beliebiger Stelle angebracht sein.

Die Ausführungsform gemäß Fig. 5 zeichnet sich weiterhin dadurch aus, daß sie auch geeignet ist, heilende Information direkt auf eine zu behandelnde Person zu übertragen. Dazu ist an der die Empfangsfläche 19 bildenden Metallplatte ein elektrisch leitender Draht 31 befestigt, der mit einem entsprechenden Akupunkturpunkt verbunden wird, der dem Organ zugeordnet ist, das behandelt werden soll. Ist das Organ auf Grund der Einwirkung eines Schadstoffes erkrankt, so genügt es, auf die Aufstellfläche 18 den Schadstoff aufzustellen und dann eine umkehrende Anordnung gemäß Fig. 3 oder gemäß Fig. 5 zu verwenden, die die Schadwirkung in eine Heilwirkung umdreht. Mit der heilenden Information wird die Person direkt behandelt. Der Behandlungsfortschritt kann von Zeit zu Zeit mit einer Meßeinrichtung 10 gemäß Fig. 1 überprüft werden. Dieselbe Behandlung kann auch dadurch ausgeführt werden, daß die zu behandelnde Person der Strahlung einer Antenne 32 ausgesetzt wird, die an der Empfangsfläche 19 befestigt ist. Der Anbringungsort des Drahtes 31 und/oder der Antenne 32 ist beliebig.

Beim Gerät gemäß den Figuren 6 und 7 bildet die Eingangsseite den Deckel 33 eines quaderförmigen Gehäuses aus Aluminium. Der Deckel wird oben in ein Gehäuseteil 34 mit U-förmigem Querschnitt eingeschoben. Die Stirnflächen des Gehäuses sind durch Stirnwände 35 mit Luftlöchern 36 verschlossen. Am Deckel 33 ist ins Gehäuseinnere hin eine Empfangsplatte 37 mit einer schlitzförmigen Ausnehmung 21 verbunden. Von der einen Endseite des Schlitzes 21 führt eine Diode zu einem Schalter 38 auf dem Deckel 33. Von der anderen Seite des Schlitzes 21 führt ein Widerstand 39 zum anderen Kontakt des Schalters 38. Beim Schließen des Schalters 38 werden die beiden Endpunkte des Schlitzes 21 über die Diode 22 und den Widerstand 39 miteinander verbunden. An der Oberseite des Deckels 33 ist eine Antenne 32 angeordnet.

Die Wirkung des Gerätes gemäß den Figuren 6 und 7 ist die folgende: Wird das Gerät z.B. im Fahrgastraum eines Fahrzeugs aufgestellt, tritt Luft durch die Luftlöcher 36 ins Gehäuseinnere und trifft auf die Empfangsplatte 37. Dadurch empfängt die Empfangsplatte 37 Signale über Wirkungen im Fahrgastraum. Das Gerät wandelt diese Wirkungen in umgekehrte Wirkungen um, wodurch die Wirkungen im Fahrgastraum neutralisiert werden.

Es ist auch möglich, daß der Deckel 33 als Platte zum Aufstellen einer Speise dient. Auf die Speise wird dann auch eine Wirkung übertragen, die zu der entgegengesetzt ist, die sie zunächst ausstrahlt. Dadurch werden auf die Speise übertragene Fremdwirkungen kompensiert.

Die Wirkungen aller Geräte sind dadurch feststellbar, daß an Personen, die den Wirkungen der Geräte direkt ausgesetzt worden sind, oder die mit diesen Geräten behandelte Medikamente oder Speisen zu sich genommen haben, vor und nach der Behandlung anhand der eingangs beschriebenen Akupunkturverfahren kontrolliert werden. Es läßt sich feststellen, daß Belastungen der Organe, die vor der Behandlung vorlagen, nach der Behandlung gelindert oder ganz beseitigt sind.

Es hat sich herausgestellt, daß es zum Übertragen der Wirkungen wichtig ist, daß die Diode 22 oder die Spannungsquelle 28 zwischen die Eingangsseite und die Ausgangsseite geschaltet sind. Anstatt eine dieser beiden Maßnahmen vorzusehen, ist es aber auch möglich, für die Eingangsseite ein Bimetall, z. B. Kupfer/Edelstahl-Bimetall zu verwenden. Die beiden Bimetallschichten liegen dabei parallel zur Plattenebene. Die Stege und die Ausgangsseite bestehen aus einem homogenen Metall.

0158310

PATENTANSPRÜCHE

1. Übertragungsgerät (16) für Elektroakupunkturwirkungen
g e k e n n z e i c h n e t   d u r c h
   (a) eine Eingangsseite (18) aus einem elektrisch leitfähigen Material,
   (b) eine Ausgangsseite (19) aus einem elektrisch leitfähigen Material und
   (c) mindestens ein die Eingangsseite mit der Ausgangs-
       seite verbindenden elektrisch leitendes Verbindungs-
       stück (27).

2. Gerät nach Anspruch 1,   d a d u r c h   g e k e n n -
   z e i c h n e t ,   daß das Verbindungsstück (27) eine
   direkte ohmsche Verbindung mit einer parallel liegenden,
   von der Eingangs- zur Ausgangsseite durchlassenden Diode (22)
   aufweist.

3. Gerät nach Anspruch 1,   d a d u r c h   g e k e n n -
   z e i c h n e t ,   daß das Verbindungsstück zwei parallele
   direkte ohmsche Verbindungen (27) mit jeweils einer parallel
   liegenden, von der Eingangs- zur Ausgangsseite durchlassenden Diode (22) aufweist.

4. Gerät nach Anspruch 1,   d a d u r c h   g e k e n n -
   z e i c h n e t ,   daß das Verbindungsstück zwei parallele
   direkte ohmsche Verbindungsleitungen (27) aufweist, denen
   eine einstellbare Gleichspannung zum Erzielen eines geringen
   Stromflusses durch die Eingangsseite und die Ausgangsseite
   zugeführt wird.

5. Gerät nach Anspruch 4,   d a d u r c h   g e k e n n -
   z e i c h n e t ,   daß die Gleichspannung durch einen
   Schalter (29) unterbrechbar ist.

6. Gerät nach Anspruch 5,   d a d u r c h   g e k e n n -
   z e i c h n e t ,   daß der Schalter (29) ein Zeitschalter
   ist.

0 4. APR. 1985

7. Gerät nach einem der vorstehenden Ansprüche, d a d u r c h
g e k e n n z e i c h n e t , daß an die Ausgangsseite (19)
ein frei endender, elektrisch leitender Draht (31) angeschlossen ist.

8. Gerät nach einem der vorstehenden Ansprüche, d a d u r c h
g e k e n n z e i c h n e t , daß die Ausgangsseite (19) an
eine beliebige Antenne (32) angeschlossen ist.

9. Gerät nach einem der vorstehenden Ansprüche, d a d u r c h
g e k e n n z e i c h n e t , daß die Ausgangsseite eine
Platte zum Aufstellen von Speisen aufweist.

Fig.1

Fig.2

Fig.3

Fig.4

Fig.5

0158310

32

35

38

22

33

37

39

21

34

**Fig.6**

37   21   22   38

32

39

33

**Fig.7**